# EUROPEAN PATENT SPECIFICATION

(11) **EP 2 285 386 B1**
(45) Date of publication and mention of the grant of the patent: **10.07.2013**
(21) Application number: 09747656.8
(22) Date of filing: 15.05.2009
(51) Int. Cl.: A61K 31/7004, A61P 25/00, A61P 25/08

(54) **Use of ribose in the treatment of restless legs syndrome**
Verwendung von Ribose zur Behandlung des Restless Legs Syndroms
Utilisation de ribose dans le traitement du syndrome des jambes sans repos

(30) Priority: 16.05.2008 US 127842 P
(43) Date of publication of application: 23.02.2011
(73) Proprietor: RiboCor, Inc., Minneapolis, MN 55428 (US)
(72) Inventor: KASUBICK, Robert, V., Red Oak, IA 51556 (US); ST. CYR, John, A., Coon Rapids, MN 55448 (US)
(74) Representative: Lucas, Brian Ronald
(86) International application number: PCT/US2009/044097
(87) International publication number: WO 2009/140575

(56) References cited:
- WO-A-01/85178
- WO-A-03/037320
- WO-A-2005/089774
- GB-A- 2 353 217
- US-A1- 2003 064 934
- US-B1- 6 294 520
- SALERNO C ET AL: "Effect of D-ribose on purine synthesis and neurological symptoms in a patient with adenylosuccinase deficiency" BIOCHIMICA ET BIOPHYSICA ACTA - MOLECULAR BASIS OF DISEASE 19990106 NL, vol. 1453, no. 1, 6 January 1999 (1999-01-06), pages 135-140, XP002545429 ISSN: 0925-4439
- SHECTERLE LINDA ET AL: "D-ribose benefits restless legs syndrome." JOURNAL OF ALTERNATIVE AND COMPLEMENTARY MEDICINE (NEW YORK, N.Y.) NOV 2008, vol. 14, no. 9, November 2008 (2008-11), pages 1165-1166, XP008111741 ISSN: 1557-7708
- SILBER MICHAEL H ET AL: "An algorithm for the management of restless legs syndrome" MAYO CLINIC PROCEEDINGS, vol. 79, no. 7, July 2004 (2004-07), pages 916-922, XP009091217 ISSN: 0025-6196

## Description

This invention relates to the treatment of restless legs syndrome.

The neurons of the central nervous system (CNS) which includes the brain and the spinal cord are sequestered against injurious chemicals by the blood brain barrier (BBB), a term used for the barrier between peripheral circulation and the neurons of the CNS. The barrier is formed by tight junctions within the brain capillary endothelial membranes and surveillances the passage of molecules, including even small molecules, and accepting those that have specific receptors which facilitate the transport of the specific molecule across the BBB. An example of the latter is the transportation of the insulin molecule, which is not true for other compounds, although lipids may cross the BBB more readily than other kinds of molecules. For example, no definitive transport receptor or mechanisms for carbohydrates in general is known; however, some carbohydrates can cross this barrier for metabolic uses, such as the molecule glucose which passes most likely via the Glu-4 transportation mechanism. It is possible for molecules or compounds that would otherwise be excluded from entrance across the BBB and influence neurons of the CNS to be "piggybacked" passively by mass action and therefore cross this guarded barrier with an acceptable molecule or compound.

Restless Legs Syndrome (RLS), also referred to as Nocturnal Myoclonus, is a comparatively mild example of a CNS/muscular alteration caused by an aberrant, uncontrollable state of neuron firing. Nevertheless, this condition has a spectrum of findings from merely an annoyance to severely interfering with daily life style or quality of life. Nocturnal RLS causes restless sleep patterns that must be distinguished clinically from other conditions, such as sleep apnea. First described by Ekborn in 1945 (as described, for example, by Pichler et al., Clinical Genetics (2008) 73(4):297-305), it is a neurological disorder characterized by unpleasant sensations and pain, predominantly in the lower extremities at rest, accompanied by an uncontrollable urge for movement for relief. The symptoms are commonly worst at night with accompanying sleep disturbances (Cotter et al., Therapeutics and Clinical Risk Management (2006) 2(4): 465-75). Furthermore, many individuals afflicted with RLS have difficulties with their daily activities, including a mental state of discomfort. The afflicted individuals have therapeutic options, such as dopaminergic, opioid or anticonvulsive compounds, to alleviate their symptoms. These substances have untoward side effects and therefore, many search for other remedies with less adverse reactions, such as alternative natural ingredients. The quest to find a universal, well-tolerated therapy for RLS continues. Although there is no known etiology for RLS, it has been observed that individuals with unhealthy eating habits and poor exercise activities are more likely to develop this condition.

This condition may have a genetic basis, possibly an autosomal dominant trait, although the nature of the genetic defect is unknown, the familial occurrence of this condition is approximately 67%, with women more susceptible than men, which supports the suspected genetic basis. Primary idiopathic RLS usually begins in the early 40's, but has been known in infants, RLS episodes may disappear for months or years. Secondary RLS can usually be traced to specific medical conditions or the use of certain medications, such as anti-nausea drugs, antihistamines, anti- depressive agents, anti-psychotic and anti-seizure drugs. The underlying mechanism for this condition has not yet been established, although theories have been proposed to focus on dopamine and iron systems.

A diagnostic test to identify this condition is missing and therefore the diagnosis is usually made by history, symptoms, and exclusion. The following clinical criteria have been recognized and are commonly used for this diagnosis: (1) the desire to move the limbs, often associated with paresthesias or synesthesias; (2) symptoms that are worse or present only during rest and are partially or temporarily relieved by activity; (3) motor restlessness; and (4) nocturnal worsening of symptoms. RLS affects between 6-15% of the adult populations with an estimated 12 million individuals affected with RLS in the United States. Many researchers believe that this estimate is low due to under diagnoses or misdiagnoses. Many individuals with RLS report socio-economic problems affecting their employment, personal relationships and activities of daily living, which are most likely associated to a lack of restful sleep, chronic exhaustion and inability to focus.

Michael H Silber et al., Mayo Clinic Proceedings, 79(7), 916-922 (July 2004) summarises treatment options for different levels of restless legs syndrome and discusses carbidopa/levodopa, dopamine agonists in general, opioids and benzodiazepines as well as magnesium and iron.

The pentose sugar D-Ribose has been extensively studied in skeletal and cardiac muscle with known benefits. It is theorized that D-Ribose accelerates the resynthesis of ATP through the pentose phosphate salvage pathway. For example, it has been shown that D-Ribose enhances cardiac recovery, e.g., U.S. Patent Application Publication No. 2004/0087515 A1 (Butler et al.) and raises the power output of exercising humans, e.g., U.S. Patent No. 6,159,942 (St. Cyr et al.). These known results confirm the theory that D-Ribose plays an important role in achieving a more ideal cellular ATP state. For adequate levels of ATP are necessary to maintain cellular integrity and function. While it is known that the brain, like muscle, is an organ with high energy demands, nothing is known about the role of D-Ribose in the brain or whether D-Ribose readily transgresses the BBB. In fact, D-Ribose has been used to improve energy levels in normal individuals and cardiac patients for some time, but has not been reported to have an effect in this group of patients who suffer from symptoms due to aberrant motor neuron firing. GB-A-2353217 discloses ribose for the treatment of fibromyalgia.

In one embodiment the present disclosure provides D-ribose for administration to a human patient for treating restless legs syndrome.

Other features are set out in the accompanying claims to which attention is directed. For example, in human patients suffering from symptoms of restless legs syndrome, two 5 gram doses of D-Ribose, taken morning and noon, followed by a dose of eight grams of D-Ribose can be sufficient to lessen the symptoms of RLS.

D-Ribose may be administered in the form of powders, lozenges, capsules or may be incorporated in a liquid drink. Time-release tablets or capsules may maintain the serum levels to the required level when taken fewer times a day.

The infusion of D-ribose can also involve the use of an infusible pump apparatus. A pump device can deliver drugs, nutrients, or biologies, even at low doses at a constant or controllable rate; thereby maintaining a relatively constant serum level of the infusible compound or molecule without interruption. An infusion catheter is commonly threaded into a central vein with the other end either exiting from the skin to an external pump, which the pump can be attached to the person for ease of mobility or to a stationary pump. The concept of an implantable pump placed subcutaneously is actively being pursued, which would require its reservoir to be filled from time to time, sterilely through the skin.

Infusible pumps are spring-operated or elastomeric. Spring-operated pumps use mechanical positive-pressure and are commonly used for intermittent antibiotic therapy, chemotherapy and analgesic therapies. The use of elastomeric pump for continuous delivery of a solution is growing in popularity. These pumps, commonly used for drug therapies, are lightweight, easy to use, easily portable, and use a positive pressure system of delivering a fluid at a constant rate for accuracy with the maintenance of a chosen serum level of the infusible solution. One of the most common uses of delivery for medication is a pump to deliver insulin; another is for the delivery of chemotherapeutic agents; and yet another is used for delivery of pain medication. Such pumps would be especially useful for delivering pyrogen-free D-Ribose to those patients who may require continuously high serum levels of D-Ribose for the control of symptoms.

As used herein, "a," "an," "the," and "at least one" are used interchangeably and mean one or more than one.

As used herein, the term "comprising," which is synonymous with "including" or "containing," is inclusive, open-ended, and does not exclude additional un-recited elements or method steps.

In one embodiment, the present disclosure provides D-ribose for use in a method for treating a human patient having restless legs syndrome. The method includes administering (e.g.,orally) to the patient in need thereof a therapeutically effective amount of a composition including D-ribose. It should be understood that the composition including D-ribose is not a cure for restless legs syndrome, but is expected to at least partially ameliorate symptoms at therapeutically effective doses. Thus, for embodiments in which the human patient has restless legs syndrome, a therapeutically effective amount of the composition means an amount which, when administered to the patient, results in the patient experiencing at least partial relief from the symptoms. In preferred embodiments, the patient can experience substantial or even total relief from the symptoms.

For certain aspects in which the human patient has restless legs syndrome, the composition including D-ribose can be administered for at least one week, at least 3 weeks, or longer (e.g., chronic administration). The therapeutically effective amount of the composition may include 4 to 50 grams of D-ribose per day. The therapeutically effective amount of the composition may be administered in 1 to 10 doses per day, 1 to 4 doses per day, or three times a day. The D- ribose content of each dose of the composition can be calculated from the desired total daily dose of D-ribose and the number of desired doses per day, and can be any convenient size (e.g., 1 gram, 1.5 grams, 2 grams, 5 grams, 6 grams of D-ribose per dose, or even higher). In some embodiments, administering the therapeutically effective amount of the composition includes administering five grams of D-ribose three times a day. In certain embodiments, administering the therapeutically effective amount of the composition consists essentially of administering five grams of D-ribose three times a day (e.g., administering a composition that can include inert and other ingredients that are not substantially active towards treating RLS). In other certain embodiments, administering the therapeutically effective amount of the composition consists of administering five grams of D-ribose three times a day (e.g., no other ingredients administered). In some embodiments the administered composition does not include folic acid and/or folate. In certain embodiments, the administered composition does not include any of vitamin B6, vitiamin B 12, folic acid, or folate. In other certain embodiments, the administered composition does not include a B group vitamin. In other certain embodiment, the administered composition does not include one or more of proteins, fatty acids, or lipids.

D-Ribose is found at low levels in the diet and normal serum levels are approximately 2 mg/dl. It has been found that D-Ribose is rapidly cleared from the blood, either by renal excretion, metabolic utilization, or conversion to D-Glucose. When infused for short periods at the concentrations of 60 mg/kg and 100 mg/kg, D-Ribose levels in the serum rose, in each case, to approximately 25 mg/dl. These doses of D-Ribose, for a 60 kilogram man, are 3.6 and 5 grams, respectively. Doses for larger or smaller size humans can be calculated based on their mass, and are typically 1 to 10 grams. It has been determined that the half-life of D-Ribose in the serum was 11 minutes and 16 minutes, respectively. At higher oral doses of a constant consumption of D-Ribose, four hours of 83.2 or 166.7 mg/kg per hour, can likewise increase the serum D-Ribose level to 29.25 mg/dl and 35 mg/dl, respectively. Intravenous administration of high doses of D-Ribose can elevate the serum levels to as high as 60 mg/dl, while a further increase in dose to 222.2 mg/kg can produce a serum level as high as 86 mg/dl. See, for example, Gross et al., Klinische Wochenschrift (1991) 69:31-36.

It has long been known that D-Ribose supplementation can reflect a decrease in measured serum glucose levels. Previous studies have substantiated this finding, including the above mentioned investigation, that confirmed this premise, and furthermore, this decline in serum glucose reaches an ultimate lower level at a 25% drop, regardless of the measured serum level of D-Ribose. Therefore, when doses of D-Ribose higher than 5 grams are taken, the patient is advised to co-ingest a source of glucose, such as a fruit drink or candy.

The following non-limiting example is provided for illustrative purposes.

### Example - Restless Legs Syndrome - Case study example

We present a report on two familial males with RLS in which the supplementation of D-ribose produced a benefit in lessening the occurrence and severity of their daily symptoms. Both affected male individuals were from a positive family prevalence, three generations carrying the diagnosis of RLS. These males involved the latter two generations, father and son, ages 71 and 47, respectively. Both men had no underlying medical conditions. Their symptoms of RLS date back for decades and presently have progressed to the moderate to severe discomfort stage, mainly experienced during sedentary periods throughout the day with exacerbation of symptoms during nocturnal sleep.

Both individuals were introduced to D-ribose. To evaluate any potential benefits of D-ribose in RLS, each individual orally consumed D-ribose (five grams per dose) daily at different trial dosing regimens. Each trial dosing regimen lasted for three weeks with a two week interval washout between dosing regimens. The initial regimen involved a single dose of oral D- ribose consumed only at breakfast. The second dosing regimen involved consumption of oral D-ribose at breakfast and lunch. In the last dosing regiment, oral D-ribose was taken with all meals, breakfast, lunch and dinner. A diary pertaining to the documentation and severity of RLS symptoms was compiled.

During the regimen interval, both men reported a general feeling of more energy and less fatigue, most notably after exercise without any significant changes in their symptoms of RLS. However, with the increase in the daily oral dose of D-ribose in the second regimen, their common leg twitching events and the urge to move during the day was reduced for one of the subjects and rarely present in the other. Both still experienced the unpleasant sensations during the night. However, during the final dosing regimen with an increase in the daily oral dose of D-ribose there was total elimination in their diurnal symptoms and the nocturnal symptoms were of a much lesser degree and had a later occurrence.

The two week washout period between stages was designed to observe any return or worsening of their symptoms. Within 48 hours after the cessation of oral D-ribose, both men experienced the return in their clinical RLS symptoms during the day and early evening hours of sleep, which were comparable to their state prior to D-ribose supplementation. Following completion of all dosing regimens of oral D-ribose and with the final wash out period, both males elected to re-start oral dosing of D-ribose at 5 grams/doses three times a day. Both males reported that while D-ribose did not totally eliminate their discomfort, the severity and onset of symptoms affecting their quality of life was substantially improved with D-ribose without any adverse reactions.

Based on the above oral pharmacokinetic information, at a 15 minute half-life, the serum D-Ribose levels would drop to near baseline of two mg/dl within an hour, with residual relief lasting several hours. Serum D-ribose levels should be maintained long enough to establish an effect that will persist after the serum D-ribose level is back to baseline levels. The effect in the brain does not persist as long as in the muscles, possibly due to the high levels of energy consumed by the brain throughout the day. Typically, approximately a third of the cardiac output is used to supply energy to the brain.

Further studies are planned to substantiate the findings of the above case study. It is possible that more frequent dosing may further improve results. D-ribose lozenges or gels may make frequent dosing more convenient than dosing at meals. A controlled release product may be most beneficial, especially in relieving the nocturnal symptoms.

The benefit of D-ribose in replenishing ATP levels has been substantiated in numerous studies in healthy individuals undergoing high levels of exercise, and in disease states or conditions of ischemia and hypoxia. Nothing has been known of the effect of D-ribose supplementation on neurological conditions. In the case of RLS, it might be said that a benefit of D-ribose is counterintuitive, since the individuals are not exercising and in fact, exercise relieves the symptoms. The well-known role of D-ribose in replenishing muscle ATP would not seem to be operative.

The most cogent observation from this small study of RLS is that there is very rapid turnover, with washout possibly occurring daily between doses and complete in 48 hours after ceasing D-ribose supplementation. In other studies, such as that of the '942 patent, it has been found that washout requires a minimum of one week and preferably two weeks for the D-Ribose effect to dissipate. Not wishing to be bound by theory, the effect may be due to passage of the D-Ribose into the brain, therein raising ATP levels and improving brain function and that D-Ribose penetrates the BBB only in small amounts, possibly co-transported with glucose through mass action when the serum levels of D-Ribose are high. Therefore, further studies on neurological diseases will employ more frequent administration of D-Ribose or preferably, time-release capsules. D-Ribose has been awarded GRAS status by the United States Food and Drug Administration, is readily administered orally and has no side effects, providing the oral dosage is kept below eight grams at a time, no more often than every two hours. It should be emphasized that this nutritional support is not a cure for the neurological diseases, but is expected to ameliorate symptoms.

## Claims

1. D-ribose for administration to a human patient for use in treating restless legs syndrome.

2. The ribose for use in treating restless legs syndrome as defined in claim 1, for administration in an amount of five grams three times per day.

3. The ribose for use in treating restless legs syndrome as defined in claim 2 for oral administration.

## Patentansprüche

1. D-Ribose zur Verabreichung an einen Human-Patienten für die Verwendung bei der Behandlung des Restless-Legs-Syndroms.

2. Ribose für die Verwendung bei der Behandlung des Restless-Legs-Syndroms nach Anspruch 1 zur Verabreichung in einer Menge von dreimal 5 g/Tag.

3. Ribose für die Verwendung bei der Behandlung des Restless-Legs-Syndroms nach Anspruch 2 für die orale Verabreichung.

## Revendications

1. D-ribose pour une administration à un patient humain destiné à être utilisé pour traiter le syndrome des jambes sans repos.

2. Ribose destiné à être utilisé pour traiter le syndrome des jambes sans repos tel que défini dans la revendication 1, pour une administration dans une quantité de cinq grammes trois fois par jour.

3. Ribose destiné à être utilisé pour traiter le syndrome des jambes sans repos selon la revendication 2 pour une administration orale.
